Europäisches Patentamt

European Patent Office

Office européen des brevets

(11) Veröffentlichungsnummer: **0 342 357**

**A2**

(12) **EUROPÄISCHE PATENTANMELDUNG**

(21) Anmeldenummer: 89106676.3

(22) Anmeldetag: 14.04.89

(51) Int. Cl.⁴: **B01J 14/00 , B01D 3/00 ,**
**C07C 67/02 , C07C 67/08 ,**
**C07C 69/22 , C07C 69/58 ,**
**C07C 69/60 , C07C 69/80**

Die Bezeichnung der Erfindung wurde geändert (Richtlinien für die Prüfung im EPA, A-III, 7.3).

(30) Priorität: 22.04.88 DE 3813612
03.08.88 DE 3826320

(43) Veröffentlichungstag der Anmeldung:
23.11.89 Patentblatt 89/47

(84) Benannte Vertragsstaaten:
AT BE CH DE ES FR GB IT LI NL SE

(71) Anmelder: Henkel Kommanditgesellschaft auf Aktien
Postfach 1100 Henkelstrasse 67
D-4000 Düsseldorf 1(DE)

(72) Erfinder: Büttgen, Karl-Heinz

Breite Strasse 57
D-5014 Karpen-Sindorf(DE)
Erfinder: Gutsche, Bernhard, Dr.
Lessingstrasse 5 a
D-4010 Hilden(DE)
Erfinder: Hommers, Friedrich, Dr.
Am Falder 53
D-4000 Düsseldorf 13(DE)
Erfinder: Johannisbauer, Wilhelm, Dr.
Erich-Kästner-Strasse 26
D-4006 Erkrath 1(DE)
Erfinder: Peukert, Eberhard
Dürerweg 15
D-4010 Hilden(DE)
Erfinder: Sedelies, Reinhold, Dr.
Backes Kamp 39
D-4030 Ratingen 8(DE)

(54) **Vorrichtung, Anwendung und Verfahren zum diskontinuierlichen Führen einer Gleichgewichtsreaktion.**

(57) Die Erfindung betrifft ein diskontinuierliches Verfahren zum Führen einer bei erhöhter Temperatur ablaufenden Gleichgewichtsreaktion eines zumindest bei Reaktionstemperatur flüssigen oder als Suspension vorliegenden Reaktionsgemisches, wobei zum Erreichen des Endumsatzes während der Reaktion ein Reaktionsprodukt aus dem Ansatz als gasförmige Phase entfernt werden muß. Um ohne thermische Schädigung der Produkte und ohne unerwünschte Neben- und Folgereaktionen eine größere Raum-Zeitausbeute als bei den bekannten Verfahren und Vorrichtungen zu ermöglichen, wird vorgeschlagen, daß zum Entfernen dieses Reaktionsprodukts das Reaktionsgemisch während der Reaktion durch eine vom Reaktor getrennte Einrichtung gefördert wird, in der das Reaktionsgemisch eine größere Flüssigkeits-Gas-Phasengrenzfläche als im Reaktor hat und durch die das Reaktionsgemisch kontinuierlich gefördert wird. Die Erfindung betrifft ferner eine Vorrichtung zum diskontinuierlichen Herstellen von thermisch empfindlichen Produkten bei erhöhten Temperaturen mit einem Reaktor und einer Heizeinrichtung. Um die genannte Aufgabe zu lösen, wird vorgeschlagen, daß die Heizeinrichtung als wenigstens ein außerhalb des Reaktors angeordneter, an diesen angeschlossener Wärmeüberträger ausgebildet ist und daß der Reaktor, eine Umwälzpumpe und der Wärmeüberträger in Reihe im Kreis geschaltet sind, und daß der Wärmeüberträger derart ausgebildet, daß in ihm das Reaktionsgemisch eine größere Flüssigkeits-Gas-Phasengrenzfläche als im Reaktor hat.

Fig. 1

## Diskontinuierliches Verfahren zum Führen einer Gleichgewichtsreaktion, Verwendung dieses Verfahrens und Vorrichtung zum diskontinuierlichen Herstellen thermisch empfindlicher Produkte

Die Erfindung betrifft zum einen ein diskontinuierliches Verfahren zum Führen einer bei erhöhter Temperatur ablaufenden Gleichgewichtsreaktion eines zumindest bei Reaktionstemperatur flüssigen oder als Suspension vorliegenden Reaktionsgemisches, wobei zum Erreichen des Endumsatzes während der Reaktion ein Reaktionsprodukt aus dem Ansatz als gasförmige Phase entfernt werden muß. Die Erfindung betrifft z. B. Veresterungen, Umesterungen, Glycerinolysen, Aminolysen, Guerbetisierungen. Dabei wird das Reaktionsgemisch, das beispielsweise bei Veresterungsreaktionen aus Fettsäuren, Alkoholen, Katalysatoren und ggf. weiteren Reaktionspartnern bestehen kann, in einen Rührkessel eingebracht und dort auf die Reaktionstemperatur, die üblicherweise im Bereich von etwa 200 bis 250 °C liegt, erwärmt. Die Beheizung erfolgt mittels außen am und/oder im Kessel angebrachter Rohrschlangen, die von einem Heizmedium, wie zum Beispiel Heizdampf durchströmt werden. An die Aufheizzeit schließen sich Reaktionszeiten im Bereich von etwa 3 bis 20 Std. an. Danach werden die Produkte abgekühlt und gereinigt.

Da die im oder am Rührreaktor installierbare Wärmeaustauschfläche durch die vorgegebene Geometrie und die vorgegebenen Maße des Rührreaktors begrenzt ist, hat auch die Raumzeitausbeute eine obere Schranke. Die Reaktion kann zum Erhöhen des Durchsatzes auch nicht bei einer höheren Temperatur durchgeführt werden, da die entstehenden und auch meist die eingesetzten Produkte thermisch empfindlich sind. Die thermische Empfindlichkeit führt zu teilweiser Zersetzung, zu Crack-Prozessen und anderen Nebenreaktionen. Auch Folgereaktionen der gewünschten Reaktionsprodukte treten in größerem Umfang auf.

Die Siedepunktserhöhung aufgrund des statischen Drucks in dem am Boden des Rührkessels befindlichen Teil des Reaktionsgemisches verhindert außerdem ein schnelles Abführen von Überhitzungen, die ebenfalls unerwünschte Neben- und Folgereaktionen bewirken.

Der Erfindung liegt die Aufgabe zugrunde, ein Verfahren der eingangs genannten Art und eine Vorrichtung zum Durchführen dieses Verfahrens zu schaffen, welche ohne eine thermische Schädigung der Produkte und ohne unerwünschte Neben- und Folgereaktionen eine größere Raumzeitausbeute als bei den bekannten Verfahren und Vorrichtungen ermöglichen.

Diese Aufgabe wird erfindungsgemäß bei einem Verfahren der eingangs genannten Art dadurch gelöst, daß zum Entfernen dieses Reaktionsprodukts das Reaktionsgemisch während der Reaktion durch eine vom Reaktor getrennte Einrichtung gefördert wird, in der das Reaktionsgemisch eine größere Flüssigkeits-Gas-Phasengrenzfläche als im Reaktor hat und durch die das Reaktionsgemisch kontinuierlich gefördert wird. Besonders vorteilhaft wirkt sich die Erfindung aus, wenn als Reaktor ein Rührkessel verwendet wird. Die Erfindung schließt ausdrücklich auch als Suspensionen vorliegende Reaktionsgemische ein, die einen oder mehrere Feststoffe enthalten.

Durch dieses Entkoppeln der Verfahrensschritte Aufheizen und/oder Kühlen und Reagieren entfällt die Begrenzung der für das Heizen und Kühlen notwendigen Wärmeaustauschflächen durch die von der Reaktion her vorgegebenen geometrischen Ausmaße des Reaktors. Unabhängig von Bauart und Abmessungen des Reaktors kann damit die Wäremaustauschfläche gestaltet und erweitert werden. Erfindungsgemäß kann damit ein für den Reaktionsablauf optimaler Reaktor verwendet werden, bei dessen Wahl nicht auf die Wärmeaustauscheigenschaften geachtet werden muß, und andererseits ein hinsichtlich der Wärmeaustauscheigenschaften optimaler Wärmeüberträger gewählt werden, bei dessen Wahl der Reaktionsablauf weitgehend unberücksichtigt bleiben darf.

Das bei der Reaktion entstandene, bei der erhöhten Temperatur flüchtige Produkt kann wegen der bezogen auf das Volumen relativ großen Phasengrenzfläche, d.h. bei einem Film wegen der geringen Filmdicke und großen Flüssigkeitsoberfläche, rasch aus dem Reaktionsgemisch entweichen. Das schnellere Entfernen dieses Reaktionsprodukts führt zu einer Verschiebung des Reaktionsgleichgewichts und damit zu einer größeren Nettoreaktionsgeschwindigkeit, also zur Verkürzung der Reaktionszeit. Durch den Wegfall des hydrostatischen Drucks im aufheizenden Aggregat sowie die kürzere Aufenthaltszeit des Reaktionsprodukts in der beheizten Zone wird das Reaktionsgemisch an den Heizflächen nicht überhitzt, und es wird eine bessere Produktqualität erreicht.

Da die Chargendauer, die sich aus der Reaktionszeit, den Aufheiz-und Abkühlzeiten sowie eventuell bei bestimmten Reaktionen aus der Zeit zum Abdestillieren von Kondensationsprodukten ergibt, zu einem sehr großen Anteil von der Größe der Wärmeaustauschflächen bestimmt ist, kann erfindungsgemäß die Gesamtchargendauer gesenkt und damit die Raumzeitausbeute erheblich vergrößert werden.

Probleme, die Raumzeitausbeute zu erhöhen, stellen sich bei Gleichgewichtsreaktionen, die bei erhöhter Temperatur ablaufen und bei denen zum Erreichen des Endumsatzes ein Reaktionsprodukt aus dem Ansatz als gasförmige Phase entfernt werden muß. Dies ist insbesondere bei Verfahren zum Herstellen von Estern aus Carbonsäuren und Alkoholen der Fall. Veresterungsreaktionen sind typische Gleichgewichtsreaktionen, bei denen größere Ausbeuten nur durch kontinuierliches Abführen von Reaktionswasser erzielt werden können. Wegen der kleinen Flüssigkeitsoberfläche der Reaktionslösung beim Einsatz eines Rührkessels ist der Stoffübergang des Veresterungswassers in die Gasphase, die laufend ausgeschleust wird, gering. Bei der daraus resultierenden relativ langen Reaktionszeit kommt es außerdem zu unerwünschten Neben- und Folgereaktionen.

Die Reaktionszeit und damit ein weiterer Anteil an der Chargendauer wird also weitgehend von der Geschwindigkeit bestimmt, mit der das Reaktionswasser abdestilliert werden kann. Wenn man die Reaktion in einem Rührkessel ablaufen läßt, muß der Nachteil in Kauf genommen werden, daß wegen des relativ großen Verhältnisses von Reaktionsvolumen zu Flüssigkeitsoberfläche die Abdestillierrate relativ niedrig und damit die Reaktionszeit relativ lang ist.

Durch die erfindungsgemäße Entkoppelung der Verfahrensschritte Reaktion und Stoffaustausch zwischen Flüssigkeit und Gas wird ermöglicht, daß die genannte getrennte Einrichtung unabhängig von den Erfordernissen der Reaktion für einen möglichst optimalen Stoffaustausch zwischen Flüssigkeit und Gas ausgelegt ist. Dazu sollte diese Einrichtung eine möglichst große spezifische Flüssigkeitsoberfläche ermöglichen.

Zum Erzielen einer großen Flüssigkeitsoberfläche muß die Flüssigkeit in Tropfen oder in dünne Filme aufgeteilt werden. Daher wird weiterhin vorgeschlagen, daß als die vom Reaktor getrennte Einrichtung eine Versprüheinrichtung, insbesondere eine Sprühkolonne, verwendet wird. Aber auch andere Verteileinrichtungen sind ohne weiteres möglich. Wichtig ist, daß solche Einrichtungen nicht nur für Flüssigkeiten geeignet sind, sondern auch das feine Versprühen von Suspensionen gestatten, so daß ein evtl. vorhandener Katalysator oder ein oder mehrere Edukte fest sein können.

Wenn man Feststoffe als dispergierte Katalysatoren oder als Reaktionsteilnehmer im erfindungsgemäßen Verfahren verwendet, muß die Verteilungseinrichtung für die Flüssigkeit besonders konstruktiv ausgestaltet sein, so daß sich kein Feststoff ablagern kann. Außerdem ist eine besondere Ausführungsform der Umwälzpumpe erforderlich, um einen Verschleiß oder ein Festsetzen der Pumpe zu vermeiden.

Eine wichtige Rolle bei der Wahl der vom Reaktor getrennten Verteilungseinrichtung spielen die Stoffeigenschaften. Bei einer relativ hohen Viskosität bietet sich z.B. eine Versprüheinrichtung an. In anderen Fällen sind Boden-, Füllkörper- sowie Fallfilm-oder Dünnschichtapparate geeignet.

Die eingangs genannte, vom Reaktor getrennte Einrichtung kann als beliebiger konventioneller Apparat ausgestaltet sein. Es kann sich z.B. um einen Rohrbündel-, Platten-, Spiral- oder Fallfilm- oder Dünnschichtwärmeaustauscher handeln. Es ist jedoch darauf zu achten, daß dieser Apparat einen hohen Wärmedurchgangskoeffizienten, eine geringe Temperaturdifferenz und eine kurze Verweilzeit aufweist, damit eine schonende Aufheizung ermöglicht wird.

Das Entfernen eines Reaktionsprodukts, z.B. des bei Veresterung entstehenden Reaktionswassers aus der flüssigen Phase wird gefördert, wenn die Einrichtung mit Unterdruck betrieben wird. Zum Senken des Partialdrucks des zu verdampfenden Wassers kann auch ein Inertgas durch die Einrichtung geführt werden.

Zweckmäßig sieht die Erfindung auch vor, daß das Reaktionsgemisch nach Abschluß der Reaktion in der genannten Einrichtung abgekühlt wird, wobei diese mit einem Kühlmedium beaufschlagt wird. Diese Verfahrensführung ist besonders einfach, da dann kein zusätzlicher Kühler notwendig ist. Durch die große Austauschoberfläche der als Wärmeüberträger arbeitenden Einrichtung und den besseren Wärmeübergang kann eine besonders schnelle Abkühlung des Reaktionsproduktes erreicht werden. Dadurch können temperaturabhängige Gleichgewichtslagen von Produkten eingefroren und unerwünschte Neben- und Folgereaktionen weitgehend bzw. zum größten Teil vermieden werden.

Nach einer weiteren vorteilhaften Ausgestaltung der Erfindung ist vorgesehen, daß nach der Reaktion das Reaktionsprodukt durch eine nachfolgende Destillation in der vom Reaktor getrennten Einrichtung gereinigt wird. In herkömmlichen Anlagen ist oft eine Überführung des Produkts nach dem Reaktionsablauf in eine externe Destillationsapparatur notwendig, um das Produkt zu reinigen. Dagegen kann nach dem erfindungsgemäßen Verfahren die Destillation bei niedriger Temperatur direkt in der genannten Einrichtung, z.B. in einem Filmverdampfer durchgeführt werden.

Insbesondere wird erfindungsgemäß das Reaktionsgemisch in der vom Reaktor getrennten Einrichtung auf Reaktionstemperatur aufgeheizt.

Vorteilhaft ist auch, wenn als diese Einrichtung ein Filmverdampfer, insbesondere ein Fallfilmverdampfer eingesetzt wird.

Das erfindungsgemäße Verfahren eignet sich nicht nur für Veresterungs-, sondern auch für Kondensations- oder Umesterungsreaktionen, insbesondere für Glycerinolyse, Aminolyse und Guerbetisierung. Dabei werden durch die Verwendung

des externen Wärmeübertragers und damit aufgrund der erheblich vergrößerten Wärmeaustauschflächen und des besseren Wärmeübertrags auf der Produktseite deutlich niedrigere Aufheiz- und Abkühlzeiten benötigt. Damit ist das Reaktionsgemisch, sowohl beim Aufheizen als auch bei der Reaktion und auch beim Abkühlen, während wesentlich kürzerer Zeiten hohen Temperaturen ausgesetzt, so daß unerwünschte Neben-und Folgereaktionen weitgehend unterdrückt werden. Durch das Vermeiden von Neben- und Folgereaktionen entfallen auch aufwendige Reinigungsoperationen wie Desodorierung und Bleichung der Produkte.

Während des Reaktionsvorganges selbst können höhere Umsatzraten erreicht werden, da im Verdampfer weitaus bessere Bedingungen für den Übergang des Reaktionswassers in die Gasphase vorliegen. Durch Ausschleusen der Gasphase wird laufend das Reaktionsprodukt, z.B. Wasser oder Alkohol, aus dem Gleichgewichtssystem entfernt und so die Rückreaktion verhindert. Die gewünschte Menge an Reaktionsprodukt wird dadurch bereits nach weitaus kürzeren Reaktionszeiten erreicht.

Das erfindungsgemäße Verfahren kann für eine Vielzahl von chemischen Reaktionen eingesetzt werden. In einer bevorzugten Ausführungsform wird das Verfahren zum Durchführen von Veresterungs-, Umesterungsreaktionen sowie Glycerinolyse, Aminolyse, Guerbetisierung eingesetzt. Bei diesen Umsetzungen handelt es sich in der Regel um Reaktionen, die zum Erzielen einer hinreichend großen Reaktionsgeschwindigkeit bei Temperaturen durchgeführt werden müssen, bei denen Ausgangsstoffe und/oder Produkte bereits thermisch labil sind sowie Folgereaktionen und Nebenreaktionen unterliegen. Des weiteren handelt es sich in den meisten Fällen um Gleichgewichtsreaktionen. Zur Erzielung einer ausreichenden Ausbeute ist es daher in den meisten Fällen notwendig, ein Reaktionsprodukt fortlaufend aus dem Reaktionssystem zu entfernen. Da dieses Ausschleusen über die Gasphase erfolgt, ist ein guter Stoffübergang der auszuschleusenden Komponente aus dem üblicherweise flüssigen Reaktionsgemisch in die Gasphase besonders wichtig.

In einer bevorzugten Ausführungsform wird das erfindungsgemäße Verfahren zum Umsetzen von Alkoholen mit 6 - 24 Kohlenstoffatomen mit Carbonsäuren mit mindestens 6 Kohlenstoffatomen eingesetzt. Diese Reaktionen werden üblicherweise bei Temperaturen oberhalb von 200 °C und mit Reaktionszeiten von 3 - 20 Stunden durchgeführt. Bei diesen Temperaturen unterliegen sowohl Ausgangsstoffe als auch Produkte bereits in größerem Umfang Zerfalls-, Neben- und Folgereaktionen.

In einer besonders bevorzugten Ausführungsform wird das erfindungsgemäße Verfahren zum Darstellen von Ölsäureoleylester aus Ölsäure und Oleylalkohol eingesetzt. In dieser besonders bevorzugten Ausführungsform kann die Reaktionszeit gegenüber dem üblichen Reaktionskessel deutlich verkürzt werden. Dies ist vor allem eine Folge des deutlich verbesserten Übergangs des Reaktionswassers in die auszuschleusende Gasphase.

In einer weiteren besonders bevorzugten Ausführungsform wird das erfindungsgemäße Verfahren zum Verestern von Isotridecylalkohol mit Stearinsäure eingesetzt. Bei dieser Reaktion wird üblicherweise der Alkohol in einem großen Überschuß eingesetzt. Im Falle dieser besonders bevorzugten Ausführungsform bietet der Einsatz eines Fallfilmverdampfers beim Abtrennen des überschüssigen Alkohols nach Ende der Reaktion besondere Vorteile.

In einer weiteren besonders bevorzugten Ausführungsform wird das erfindungsgemäße Verfahren zum Verestern von Methylcyclohexanol mit Phthalsäureanhydrid unter Bilden des Diesters eingesetzt. In diesem Falle ist der Einsatz des erfindungsgemäßen Verfahrens vor allem zur Produkterwärmung besonders vorteilhaft, da wegen der vergleichsweise geringen Heizflächenbelastung im Vergleich zum einfachen Rührkesselbetrieb eine thermische Produktschädigung weitgehend vermieden werden kann.

In einer weiteren bevorzugten Ausführungsform wird das erfindungsgemäße Verfahren für das Umsetzen von Alkoholen mit 6 bis 24 Kohlenstoffatomen mit kurzkettigen, polymerisierbaren Carbonsäuren eingesetzt.

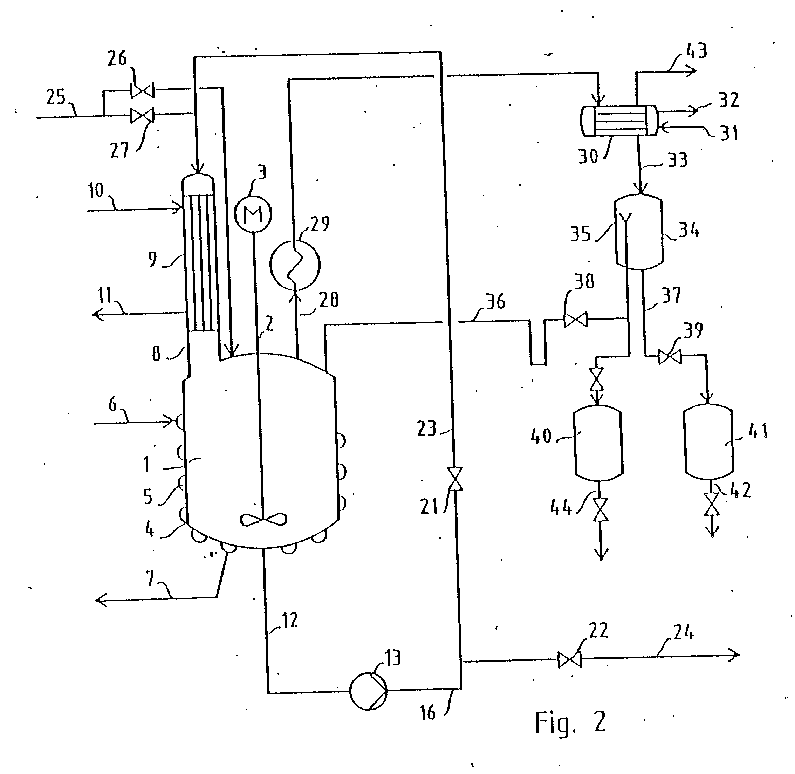

Im Falle des Umsetzens von Behenylalkohol mit Fumarsäure, einer besonders bevorzugten Ausführungsform des erfindungsgemäßen Verfahrens, kann die Reaktionszeit um etwa 40 bis 80 % gesenkt werden. Auch ist es möglich, bei Arbeiten unter vermindertem Druck bereits bei Temperaturen von etwa 180 °C ausreichende Reaktionsgeschwindigkeiten zu erzielen. Vergleichbare Vorteile werden beim Umsetzen von Fumarsäure mit einem Gemisch von Alkoholen mit im wesentlichen 8 bis 18 Kohlenstoffatomen, einer weiteren besonders bevorzugten Ausführungsform des erfindungsgemäßen Verfahrens, erzielt. In allen Fällen wurde die aus den Edukten durch Rühren erzeugte Suspension mit den ungelösten Fumarsäure-Partikeln über die Pumpe (13) und den Fallfilmverdampfer (9) entsprechend Figur 2 umgewälzt.

Die Erfindung sieht auch eine Vorrichtung zum diskontinuierlichen Herstellen von thermisch empfindlichen Produkten bei erhöhten Temperaturen mit einem Reaktor und einer Heizeinrichtung vor, die als wenigstens ein außerhalb des Reaktors angeordneter, an diesen angeschlossener Wärmeüberträger ausgebildet ist, wobei der Reaktor, eine Umwälzpumpe und der Wärmeüberträger in Reihe im Kreis geschaltet sind und der Wärmeüberträger derart ausgebildet ist, daß in ihm das Reaktionsgemisch eine größere Flüssigkeits-Gas-Phasengrenzfläche als im Reaktor hat. In dieser Vorrichtung wird die zur Verfügung stehende Gasaustauschfläche nicht durch die Größe des Reaktors beschränkt, sondern hängt nur von der Auslegung des Wärmeüberträgers ab. So kann ein wesentlich schnelleres Ausschleusen von gasförmigen Zwischen- oder Endprodukten erreicht werden und die erfindungsgemäße Entkopplung von Reagieren und Ausschleusen verwirklicht werden.

In einer besonders vorteilhaften Ausgestaltung ist der Wärmeüberträger als Filmverdampfer, insbesondere als Fallfilmverdampfer ausgebildet. Derartige Verdampfer weisen eine besonders große Austauschoberfläche und eine geringe Filmdicke des aufzuwärmenden Reaktionsgemisches auf. Damit ist eine besonders schnelle und homogene Aufheizung möglich. Für Reaktionsmischungen hoher Viskosität sind Dünnschichtverdampfer, bei denen die Filmbildung durch mechanisches Verstreichen der Reaktionslösung erfolgt, dann vorgesehen, wenn es im Fallfilmverdampfer nicht zu einer entsprechenden Filmbildung kommt. Kurzwegverdampfer eignen sich vor allem für Systeme mit schwerflüchtigen Reaktionsprodukten (z.B. Riechstoffe), bei denen ein hochreines Produkt abgetrennt werden soll.

Dabei besteht eine besonders vorteilhafte Ausgestaltung darin, daß der Filmverdampfer über einen Anschlußstutzen direkt am Reaktor angeordnet ist. Dann können nämlich die entstehenden Brüden aus dem Reaktor abgeführt werden und in diesen eintreten. Im Gegenstromverfahren können die Brüden direkt am Kopf des Verdampfers abgeführt werden. Der senkrecht auf den Reaktor aufgesetzte Fallfilmapparat kann als Rohrbündel in nahezu beliebiger Größe ausgeführt werden. Im Betrieb wird die Flüssigkeit aus dem Reaktor mit einer Pumpe auf den Kopf des Fallfilmwärmeaustauschers gefördert. Das flüssige Gemisch fließt auf der Innenseite als dünner Film herunter und in den Reaktor zurück. Dadurch ist eine nur geringe Temperaturdifferenz zwischen Produkt und Heizmedium im Außenraum des Rohrbündels notwendig. Als Folge davon werden die Produkte nur während kurzer Verweilzeiten an den beheizten Flächen thermisch beansprucht.

Sind die Ausgangsstoffe bei Umgebungstemperatur fest, so kann zum Aufschmelzen von festen Ausgangsstoffen bzw. zum Aufrechterhalten der Temperatur im Reaktor am äußeren Mantel des Reaktors eine Mantelbeheizung angeordnet sein.

Die Erfindung wurde in vielen Versuchen ausgeführt und getestet. Besonders erfolgreich verlief die Herstellung von Fumaraten und Ölsäureoleylester, bei denen die Reaktionszeit erheblich, nämlich um mehr als 50 % reduziert werden konnte. Auch bei der Herstellung anderer Produkte wurde die Gesamt-Chargenzeit verkürzt, wenn auch die Reaktionszeit in einigen Fällen die gleiche blieb.

In einer zusätzlichen vorteilhaften Ausgestaltung weist die erfindungsgemäße Vorrichtung oder Anlage eine Einrichtung mit einer größeren Flüssigkeits-Gas-Phasengrenzfläche als der Reaktor auf, an dessen Eingang der Ausgang der genannten Einrichtung angeschlossen ist. Diese Einrichtung kann eine Filmkolonne oder eine Sprühkolonne oder eine andere Apparatur zum Vergrößern der Flüssigkeitsoberfläche sein. Aus dieser Einrichtung fließt das Reaktionsgemisch, nachdem eine größere Menge an flüchtigem Nebenprodukt wie Veresterungswasser verdampft ist, in den Reaktor zurück.

Ferner kann die Einrichtung vorteilhaft innerhalb des Reaktors, aber oberhalb der kontinuierlichen Flüssigkeitsoberfläche angeordnet sein.

Besondere Vorteile hat die erfindungsgemäße Vorrichtung, wenn der Reaktor als Rührkessel ausgebildet ist.

Ausführungsbeispiele der Erfindung sind nachstehend anhand von Zeichnungen näher erläutert.

In einem Ausführungsbeispiel wurden Fumarate hergestellt. Das Ausgangsmaterial Fumarsäure liegt mit einem Schmelzpunkt von 300 $^\circ$C und einem Anteil von anfangs 30 % während der Reaktion als Feststoff vor. Die erfindungsgemäße Anlage ließ sich dennoch problemlos anfahren und betreiben, da sie für das Umwälzen und Fördern von mit Feststoff beladenen Flüssigkeiten ausgerüstet wurde. Auch am Flüssigkeitsverteiler des Fallfilmverdampfers wurden keine Produktablagerungen festgestellt.

In der erfindungsgemäßen Vorrichtung sinkt durch den verbesserten Stoffübergang die erforderliche Reaktionstemperatur bei der Fumaratherstellung von 220 $^\circ$C im reinen Rührkesselbetrieb auf etwas 180 $^\circ$C beim Beheizen mit dem Fallfilmverdampfer.

Die Erfindung, zum Beheizen eines diskontinuierlich betriebenen Reaktors eine davon getrennte Einrichtung, insbesondere einen Fallfilmverdampfer einzusetzen, hat sich bei allen untersuchten Produkten bewährt. Neben der Chargenzeitverkürzung während der Aufheizphase durch den verbesserten Wärmeübergang und die gegenüber einem mit Mantelheizung versehenen Rührkessel nahezu beliebig vergrößerbare Wärmeaustauschfläche ergaben sich bei einigen der untersuchten Produkte auch deutlich kürzere Reaktionszeiten. Die erfindungsgemäße produktschonendere Fahrweise, die durch niedrigere Reaktions- und Heizflächentemperaturen bedingt ist, ermöglicht zusätzlich eine Qualitätssteigerung bei besonders empfindlichen Produkten.

Eine Modellrechnung für eine solche erfindungsgemäße Anlage ergab ohne Berücksichtigung sonstiger produktspezifischer Vorteile während der Reaktionsphase beim Vergleich der Aufheiz- und Abkühlphase einer Anlage mit externen Wärmeaustauschern entsprechend der Erfindung gegenüber dem bekannten, einfachen, mantelbeheizten Rührkessel eine mögliche Chargenzeitverkürzung von mehr als 5 Stunden.

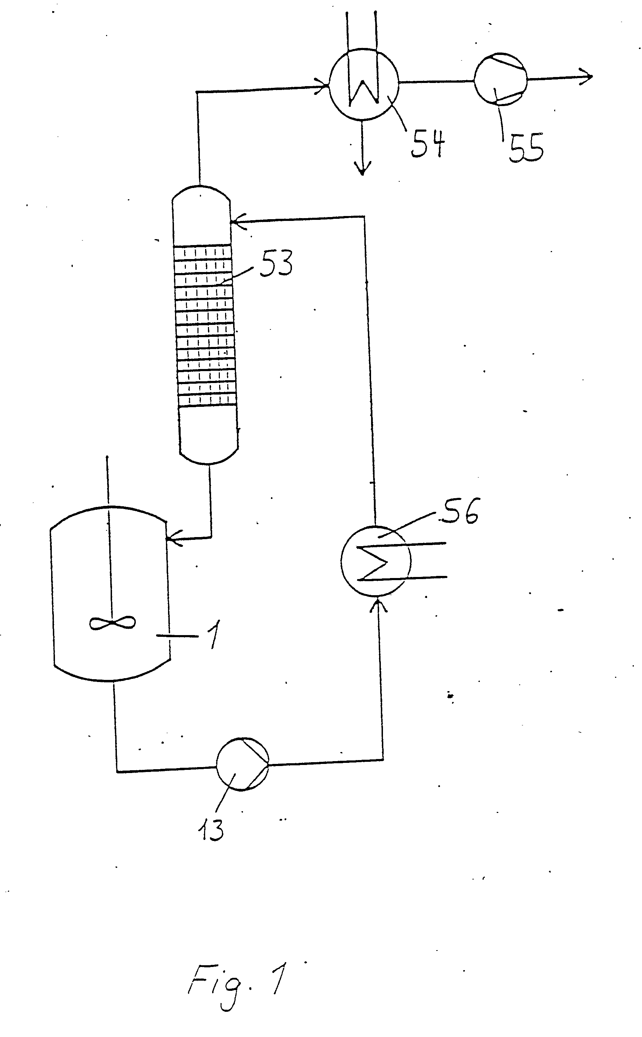

In Figur 1 ist eine der möglichen erfindungsgemäßen Vorrichtungen schematisch dargestellt. Über die Apparaturen (1), (13), (56) und (53) läuft das Reaktionsgemisch im Kreis. Das Kondensat wird über den Kondensator (54) und die Vakuumpumpe (55) abgezogen.

Aus dem Reaktor (1) wird das Reaktionsgemisch von der Umwälzpumpe (13) durch den Wärmeüberträger (56) in die Kolonne (53) gepumpt, welche eine Film- oder Sprühkolonne sein kann.

Aus der Kolonne (53) fließt oder tropft das Reaktionsgemisch, nachdem dessen flüchtige Bestandteile über den Kondensator (54) und die Vakuumpumpe (55) abgezogen worden sind, in den Reaktor (1) zurück.

In Figur 2 ist ein detaillierteres Verfahrensfließbild einer Mehrzweckreaktoranlage mit einem externen Heiz- und Kühlsystem entsprechend der Erfindung dargestellt.

Eine Mehrzweckreaktoranlage für Veresterungs- und Umesterungsprozesse oder dgl. weist einen Reaktor (1) mit Rührer (2) und Rührermotor (3) auf. Am äußeren Mantel (4) des Reaktors (1) ist eine Mantelbeheizung (5) angeordnet, durch welche über Leitungen (6) und (7) ein Heizmedium, wie Heizdampf, geführt ist.

Im oberen Bereich des Reaktors (1) ist ein Anschlußstutzen (8) angeordnet, auf den ein Fallfilmverdampfer (9) aufgesetzt ist. Dieser Fallfilmverdampfer (9) wird über Leitungen (10) und (11) mit einem Heiz- bzw. Kühlmedium versorgt.

Vom Reaktor (1) führt eine Umwälzleitung (12) weg, in der eine Förderpumpe (13) angeordnet ist. Die aus der Förderpumpe (13) geführte Leitung (16) ist über zwei Ventile (21 und 22) in eine Rückführleitung (23) und eine Produktabführleitung (24) verzweigt, wobei die Rückführleitung (23) über den Fallfilmverdampfer (9) in den Reaktor (1) zurückgeführt ist.

Eine Feedleitung (25) ist über zwei Ventile (26 und 27) in den Reaktor (1) bzw. den Fallfilmverdampfer (9) geführt.

Am Reaktor (1) ist eine Brüdenleitung (28) angeordnet, welche durch einen Wärmeaustauscher (29) geführt ist. Die Brüdenleitung (28) mündet in einen Kondensator (30), der über Leitungen (31), (32) von einem Kühlfluid durchströmt wird. Am Austritt des Kondensators (30) ist eine Leitung (33) angeordnet, die in einen Abscheider (34) mit Trennschichtregler (35) mündet. Dabei ist an den Trennschichtregler (35) eine Rückführleitung (36) angeschlossen, die über das Ventil (38) in den Reaktor (1) zurückgeführt ist. Das Produkt kann aber auch in den Behälter (40) geleitet werden. Über die an den Kondensator (30) angeschlossene Leitung (43) kann im System Unterdruck erzeugt werden.

Am Fuß des Abscheiders (34) ist eine Leitung (37) angeordnet, die über ein Ventil (39) in den Behälter (41) mündet. Der Inhalt der Behälter (40, 41) kann dabei über die Leitungen (42, 44) abgeführt werden.

Die Ausgangsstoffe werden über die Feedleitung (25) dem Reaktor (1) zugeführt. Dabei erfolgt die Zuleitung entweder durch Öffnen des Ventils (26) direkt in den Reaktor (1), wenn feste Ausgangsstoffe vorhanden sind, oder durch Öffnen des Ventils (27) zur Vorwärmung über den Fallfilmverdampfer (9) in den Reaktor (1). Nachdem der Reaktor (1) vollständig gefüllt ist, werden die Ventile (26) bzw. (27) geschlossen. Liegen nur feste Ausgangsstoffe vor, so wird über die Mantelbeheizung (5) der Reaktor (1) erwärmt, um eine Suspension bzw. ein flüssiges Gemisch zu erhalten. Über den Rührer (2) wird dabei jederzeit eine homogene Verteilung im Reaktor (1) erreicht. Gleichzeitig wird durch die Mantelbeheizung (5) die jeweilige Reaktionstemperatur im Reaktor (1) aufrechterhalten.

Zum weiteren Erwärmen und während der Reaktionsphase wird der Ausgangsstoff von der Förderpumpe (13) über die Umwälzleitung (12) aus dem Reaktor (1) abgezogen und gelangt über die Leitung (16) und die Rückführleitung (23) in den Fallfilmverdampfer (9) und wird dort erwärmt, um über den Anschlußstutzen (8) in den Reaktor (1) zurückzugelangen.

Das Reaktionsgemisch wird anschließend etwa 5 bis 20 mal pro Stunde kontinuierlich von der Förderpumpe (13) durch die Umwälzleitung (12), Leitung (16) und Rückführleitung (23) jeweils hintereinander im Kreislauf durch den Fallfilmverdampfer (9) und den Reaktor (1) gefördert. Nach einer bestimmten Aufheizzeit entstehen dabei im Verdampfer (9) Brüden, die bei Veresterungs- und Umesterungsreaktionen, Glycerinolysen, Aminolysen und Guerbetisierung insbesondere verdampftes Wasser und Alkohol enthalten.

Die Brüden gelangen vom Fallfilmverdampfer (9) über den Reaktor (1) in die Brüdenleitung (28) und werden im Wärmeaustauscher (29) teilweise abgekühlt, so daß der flüssige Bestandteil in den Reaktor (1) zurückfließt, während die restlichen Brüden in den Kondensator (30) geführt und in diesem abgekühlt werden. Die verflüssigten Brüden fließen aus dem Kondensator (30) über die Leitung (33) in den Abscheider (34). Dabei gelangt die leichtere Phase über den Trennschichtregler (35) in die Rückführleitung (36) und über diese in den Reaktor (1) zurück oder kann bei Bedarf in den Behälter (40) abgelassen werden. Die schwere, wasserhaltige Phase dagegen wird über die Leitung (37) in den Behälter (41) abgeführt und kann über die Leitung (42) entnommen werden.

Das vom Reaktor (1) über die Umwälzleitung (12), Leitung (16) und Rückführleitung (23) in den Fallfilmverdampfer (9) und zurück in den Reaktor (1) umgewälzte Reaktionsprodukt wird solange im Fallfilmverdampfer (9) beheizt, bis die gewünschten Reaktionen vollständig abgelaufen sind. Während dieser Zeit wird der Fallfilmverdampfer (9) mit einem Heizmedium beaufschlagt. Anschließend wird der Fallfilmverdampfer (9) von Heizen auf Kühlen umgeschaltet, wobei der Fallfilmverdampfer (9) mit einem Kühlmedium beaufschlagt wird. Auch während der Kühlung im Fallfilmverdampfer (9) wird das Reaktionsprodukt zuerst vom Reaktor (1) über die Umwälzleitung (12), Leitung (16) und Rückführleitung (23) in den Fallfilmverdampfer (9) und den Reaktor (1) zurück umgewälzt.

Durch die Umlaufkühlung im Fallfilmverdampfer (9) findet eine sehr schnelle Abkühlung des Reaktionsgemisches statt, so daß Neben- und Folgereaktionen weitestgehend vermieden werden. Je nach der erforderlichen Abkühlung des Reaktionsgemisches wird dabei der Kühlkreislauf mehrfach durchlaufen.

Nach ausreichender Abkühlung wird das Ventil (21) geschlossen und das Ventil (22) geöffnet, so daß das Reaktionsprodukt über die Leitung (24) entnommen werden kann. Wenn der Reaktor (1) vollständig entleert ist, kann dann über die Feedleitung (25) eine neue Charge zugeführt werden.

Natürlich ist die Erfindung nicht auf die in den Zeichnungen dargestellten Ausführungsbeispiele beschränkt. Weitere Ausgestaltungen sind möglich, ohne den Grundgedanken zu verlassen. So kann anstelle des Fallfilmverdampfers (9) auch ein Dünnschicht- oder Kurzwegverdampfer eingesetzt werden. Ebenso kann die Energie über andere Wärmeüberträger-Bauarten eingetragen werden, wenn am Reaktor eine Einrichtung zum Erzeugen großer Flüssigkeits/Gas-Phasengrenzflächen installiert ist, wie z.B. eine Sprüheinrichtung. Der Verdampfer (9) kann auch in Gegenstromfahrweise betrieben werden. Bei dieser Betriebsart werden die Brüden am Verdampferkopf abgezogen, wobei das flüssige Reaktionsprodukt in den Reaktor zurückläuft.

Alternativ ist es auch möglich, daß das Reaktionsgemisch nach der Reaktion vom Reaktor in einen Platten-, Rohrbündel- oder Spiralwärmeaustauscher gefördert und in diesem abgekühlt wird. Dadurch läßt sich z.B. eine schnelle Abkühlung von bei tiefen Temperaturen sehr zäh werdenden Produkten erreichen. Der Einsatz eines solchen Kühlers ist z.B. auch dann sinnvoll, wenn der Verdampfer mit mit Wasser nicht mischbaren Heizmedien, wie Thermalöl, betrieben wird.

Weiterhin kann nach dem Ablauf der gewünschten Reaktion das Reaktionsprodukt direkt im Verdampfer destillativ gereinigt werden.

**Ansprüche**

1. Diskontinuierliches Verfahren zum Führen einer bei erhöhter Temperatur ablaufenden Gleichgewichtsreaktion eines zumindest bei Reaktionstemperatur flüssigen oder als Suspension vorliegenden Reaktionsgemisches, wobei zum Erreichen des Endumsatzes während der Reaktion ein Reaktionsprodukt aus dem Ansatz als gasförmige Phase entfernt werden muß, dadurch gekennzeichnet, daß zum Entfernen dieses Reaktionsprodukts das Reaktionsgemisch während der Reaktion durch eine vom Reaktor (1) getrennte Einrichtung (9, 53) gefördert wird, in der das Reaktionsgemisch eine größere Flüssigkeits-Gas-Phasengrenzfläche als im Reaktor (1) hat und durch die das Reaktionsgemisch kontinuierlich gefördert wird.

2. Verfahren nach Anspruch 1, dadurch gekennzeichnet, daß als Reaktor (1) ein Rührkessel verwendet wird.

3. Verfahren nach Anspruch 1 oder 2, dadurch gekennzeichnet, daß als die vom Reaktor (1) getrennte Einrichtung eine Versprüheinrichtung, insbesondere eine Sprühkolonne (53), verwendet wird.

4. Verfahren nach einem der Ansprüche 1 bis 3, dadurch gekennzeichnet, daß die Einrichtung (9, 53) mit Unterdruck betrieben wird.

5. Verfahren nach einem der Ansprüche 1 bis 3, dadurch gekennzeichnet, daß durch die Einrichtung (9, 53) ein Inertgas geführt wird.

6. Verfahren nach einem der Ansprüche 1 bis 5, dadurch gekennzeichnet, daß nach der Reaktion das Reaktionsprodukt durch eine nachfolgende Destillation in der vom Reaktor getrennten Einrichtung (53, 56) gereinigt wird.

7. Verfahren nach einem der Ansprüche 1 bis 6, dadurch gekennzeichnet, daß das Reaktionsgemisch in der vom Reaktor (1) getrennten Einrichtung (9, 53) auf Reaktionstemperatur aufgeheizt wird.

8. Verfahren nach einem der Ansprüche 1 bis 7, dadurch gekennzeichnet, daß als die Einrichtung (9, 53) ein Filmverdampfer (9), insbesondere ein Fallfilmverdampfer (9) eingesetzt wird.

9. Verwendung eines Verfahrens nach einem der Ansprüche 1 bis 8 für Veresterungs-, Kondensations- oder Umesterungsreaktionen, insbesondere für Glycerinolyse, Aminolyse und Guerbetisierung.

10. Verwendung eines Verfahrens nach einem der Ansprüche 1 bis 9 zum Umsetzen von 6 bis 24 Kohlenstoffatome aufweisenden Alkoholen mit mindestens 6 Kohlenstoffatome aufweisenden Carbonsäuren.

11. Verwendung eines Verfahrens nach einem der Ansprüche 1 bis 9 zum Herstellen von Fumaraten aus Fumarsäure und Alkohol, insbesondere Behenylalkohol.

12. Verwendung eines Verfahrens nach einem der Ansprüche 1 bis 9 zum Herstellen von Ölsäureoleylester aus Ölsäure und Oleylalkohol.

13. Verwendung eines Verfahrens nach einem der Ansprüche 1 bis 9 zum Verestern von Isotridecylalkohol mit Stearinsäure.

14. Verwendung eines Verfahrens nach einem der Ansprüche 1 bis 9 zum Verestern von Methylcyclohexanol mit Phthalsäureanhydrid zum Herstellen des Diesters.

15. Vorrichtung zum diskontinuierlichen Herstellen von thermisch empfindlichen Produkten bei erhöhten Temperaturen mit einem Reaktor (1) und einer Heizeinrichtung (56), dadurch gekennzeichnet, daß die Heizeinrichtung als wenigstens ein außerhalb des Reaktors (1) angeordneter, an diesen angeschlossener Wärmeüberträger (56,9) ausgebildet ist und daß der Reaktor (1), eine Umwälzpumpe (13) und der Wärmeüberträger (56,9) in Reihe im Kreis geschaltet sind, und daß der Wärmeüberträger (56,9) derart ausgebildet ist, daß in ihm das Reaktionsgemisch eine größere Flüssigkeits-Gas-Phasengrenzfläche als im Reaktor (1) hat.

16. Vorrichtung nach Anspruch 15, dadurch gekennzeichnet, daß der Wärmeüberträger (56) als Filmverdampfer, insbesondere als Fallfilmverdampfer (9) ausgebildet ist.

17. Vorrichtung nach Anspruch 16, dadurch gekennzeichnet, daß der Filmverdampfer (9) über einen Anschlußstutzen (8) direkt am Reaktor (1) angeordnet ist.

18. Vorrichtung nach einem der Ansprüche 15 bis 17, gekennzeichnet durch eine Einrichtung (53) mit einer größeren Flüssigkeits-Gas-Phasengrenzfläche als der Reaktor, an dessen Eingang der Ausgang der Einrichtung (53) angeschlossen ist.

19. Vorrichtung nach Anspruch 18, dadurch gekennzeichnet, daß die Einrichtung (53) innerhalb des Reaktors (1), aber oberhalb der kontinuierlichen Flüssigkeitsoberfläche angeordnet ist.

20. Vorrichtung nach einem der Ansprüche 15 bis 19, dadurch gekennzeichnet, daß der Reaktor (1) als Rührkessel ausgebildet ist.

Fig. 1

Fig. 2